(19) Europäisches Patentamt
European Patent Office
Office européen des brevets

(11) **EP 3 000 451 B1**

(12) **EUROPÄISCHE PATENTSCHRIFT**

(45) Veröffentlichungstag und Bekanntmachung des Hinweises auf die Patenterteilung:
**11.04.2018 Patentblatt 2018/15**

(51) Int Cl.:
*A61F 13/62* (2006.01)     *D04H 1/4282* (2012.01)
*D04H 1/4291* (2012.01)     *D04H 1/485* (2012.01)
*D04H 1/544* (2012.01)

(21) Anmeldenummer: **15178833.8**

(22) Anmeldetag: **29.07.2015**

(54) **DEHNBARER VLIESSTOFF**

STRETCHABLE NONWOVEN FABRIC

NON-TISSE EXTENSIBLE

(84) Benannte Vertragsstaaten:
**DE FR IT**

(30) Priorität: **23.09.2014 DE 102014013808**

(43) Veröffentlichungstag der Anmeldung:
**30.03.2016 Patentblatt 2016/13**

(73) Patentinhaber: **Sandler AG**
**95126 Schwarzenbach/Saale (DE)**

(72) Erfinder:
• **Die Erfinder haben auf ihr Recht verzichtet, als solche bekannt gemacht zu werden.**

(56) Entgegenhaltungen:
**EP-A1- 2 228 209      EP-A2- 2 008 565**
**WO-A1-03/007864      WO-A1-2008/066417**

**Beschreibung**

Die vorliegende Erfindung beschäftigt sich mit dehnbaren Vliesstoffen, wie sie beispielsweise als Komponenten in elastischen Verschlusssystemen für persönliche Hygieneartikel wie beispielsweise Windeln eingesetzt werden.

Derartige Verschlusssysteme umfassen dabei beispielsweise ein auf dem Windelbund sitzenden fasriges Material, die sogenannte Landing Zone, und als Gegenstück dazu das sogenannten Windelohr. Das Windelohr besteht dabei nach dem Stand der Technik aus einem mehrlagigen Laminat, welches auf der der Landing Zone zugewandten Seite ein Hakenmaterial aufweist, das sich beim Schließen der Windel in der Landing Zone verhakt.

Um den Tragekomfort und die Passform zu erhöhen enthalten diese Laminate, welche für Windelohren eingesetzt werden, elastische Komponenten, häufig Folien, die nach dem Schließen der Windel durch die Rückstellkraft des Elastikanteils eine Anpassung des Windelbundes an die Körperform ermöglichen. Um dem Benutzer grifflich ein ansprechendes Gefühl zu geben, sind die elastischen Komponenten mit textilen Deckmaterialien abgedeckt.

Untersuchungen haben ergeben, dass der Benutzer beim Schließen einer Windeln am Windelohr eine Kraft im Bereich von max. 10N aufbaut. Die dabei übliche Dehnungsbeanspruchung liegt im Bereich von 0-100%. Nach Wegfall der Dehnbeanspruchung muss sich das Windelohr aufgrund der Elastizität weitestgehend wieder zurückstellen. Dieser Bereich zwischen 0 bis 10N Belastung und 0 bis 100% Dehnung wird auch als funktioneller Bereich bezeichnet.

Dafür geeignete Materialien bestehen zumeist aus Laminaten, bei welchen eine elastische Schicht zumindest auf einer Seite mit einer Deckschicht abgedeckt ist. Der Verbund zwischen den Schichten kann dabei mittels Verklebung oder auch thermischer Laminierung erfolgen.

Für eine geeignete Deckschicht ergeben sind daher folgende Anforderungen:

    a. Geringe Steigung der Kraft-Dehnungs-Kurve im funktionellen Bereich
    b. Keine Breitenänderung bei der Verarbeitung
    c. Wenige Arbeitsschritte bei der Windel-Ohr-Herstellung
    d. Kein Abflusen von Fasermaterial während der Benutzung
    e. Geringe Staubbildung bei der Verarbeitung.
    f. Stoppfunktion zur Verhinderung einer Überdehnung
    g. Wirtschaftliche Verarbeitbarkeit

Die prinzipiell am Markt eingeführten System sind in dem Artikel "Performance and Fit", der Autoren Dr.Hornfeck und Bernhuber, erschienen auf den Seiten 56 bis 62 der Januar-Ausgabe 2013, der Zeitschrift "Nonwoven Industry" beschrieben. Das Dokument ist unter der Internet-Adresse http://www.nonwovens-industry.com/issues/2013-01/view features/performance-and-fit/ abrufbar.

Deckschichten können aus thermisch kalanderverfestigten Stapelfaservliesen bestehen. Gattungsbildend ist hier die EP2069141 B1.

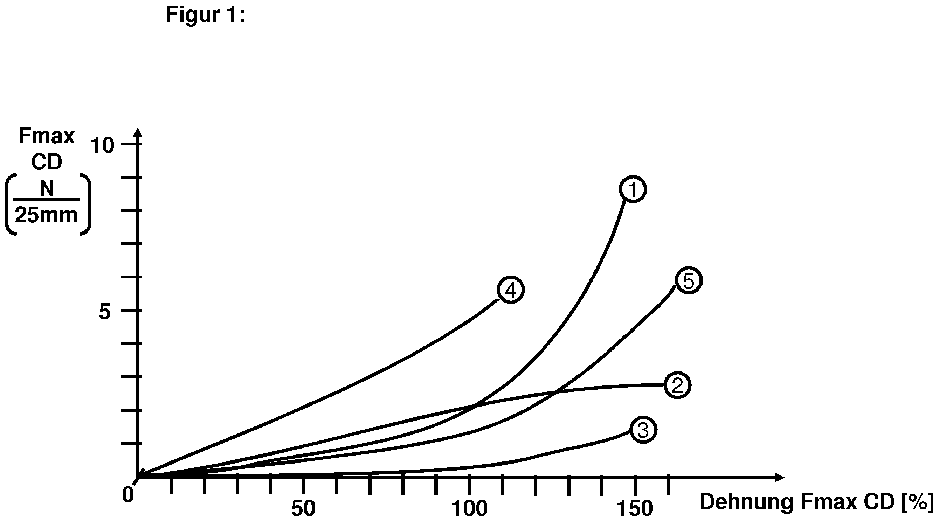

Kennzeichnend für derartige Stapelfaservliese ist eine Kraft-Dehnungsverhalten wie in Figur 1, Kurve 2 dargestellt. Die Verfestigungsfläche am Kalander liegt zwischen 8 und 22%. Durch diese thermische Verfestigung wird der der Verfestigungsfläche entsprechende Anteil von Fasern im Vlies miteinander verschmolzen und daher im Vliesverbund fixiert. Derartige Vliese haben trotz guter Dehnbarkeit in Querrichtung eine ausreichende Stabilität in Längsrichtung, sodass Warenspannungsschwankungen in Längsrichtung nur wenig Einfluss auf die Breite haben. Derartige Vliesstoffe sind auch dünn genug um große Rollenlängen herzustellen, was sich positiv auf die Effizienz bei der Weiterverarbeitung auswirkt.

Nachteilig ist bei derartig hergestellten Stapelfaservliesen, dass diese im funktionellen Bereich des Windelohrs eine im Vergleich zur Elastikkomponente zu steile Kraft-Dehnungskurve aufbauen, was der Anwender als negativ empfindet.

Üblicherweise werden daher Laminate, die thermisch kalanderverfestigte Stapelfaservliese verwenden, in einem zusätzlichen Arbeitsschritt aktiviert. Diese Aktivierung stellt eine Überdehnung des Materials in Querrichtung mit dem Zweck dar, einen Großteil der bestehenden Bindepunkte aufzubrechen, sodass in der späteren Benutzung die zur Dehnung des Windelohrs aufzubringende Kraft möglichst gering ist. Diese Aktivierung erfolgt im laminierten Zustand, d.h. zusammen mit der Elastikkomponente. Nachteilig ist, dass dabei diese Vliese in ihrer Struktur derart geschädigt werden, sodass die das Vlies bildenden Fasern nicht mehr vollständig im Vlies gehalten werden. In der Folge können Fasern aus dem Vlies ausgetragen werden und abflusen, auch das Delaminieren ist möglich.

Bezogen auf das vorerwähnte Anforderungsprofil ergibt sich, dass thermisch kalanderverfestigte Vliesstoffe die Anforderungen b), e) und g) erfüllen, aber die anderen Anforderungen nicht.

Die EP 1921192B1 beschreibt die Verwendung von wasserstrahlverfestigten Filament- oder Stapelfaservliesstoffen für den gleichen Einsatzzweck. Aufgrund der Wasserstrahlverfestigung sind die Verfestigungsstellen der Fasern untereinander nicht starr fixiert sondern flexibel. Eine für ein derartiges Material typische Kraft-Dehnungskurve ist der Figur 1, Kurve 1 zu entnehmen. Der vorbeschriebene Aktivierungsgang kann bei der Verwendung von derartigen Vliesstoffen entfallen.

Durch die Wasserstrahlvernadelung werden die Fasern umorientiert, sodass die ursprüngliche zweidimensionale Faserausrichtung in eine dreidimensionale Faserorientierung überführt wird. Die Fasern sind daher stärker in das Vlies eingebunden, sodass die Gefahr der Staubbildung bei der Verarbeitung oder aber das Abflusen im Gebrauch minimiert wird.

Nachteilig ist bei derartig wasserstrahlverfestigten Vliesen, dass diese eine höhere Dicke als die vorerwähnten kalanderverfestigten Vliesstoffe aufweisen, in der Folge haben Rollen eine geringere Lauflänge, was einen häufigeren Rollenwechsel notwendig macht und dadurch Kosten verursacht. Des weiteren haben wasserstrahlverfestigte Vliese im Vergleich zu kalanderverfestigten Vliesen eine höhere Dehnung in Längsrichtung des Materials und sind daher schwieriger in der Verarbeitung, da bereits geringe Schwankungen im Bahnzug zu Breiteneinsprüngen führen und so Ausschuss aufgrund von Minderbreite erzeugt wird.

Das Patent WO 03 / 007864 A1 beschreibt neben den im EP 1921192B1 wasserstrahlverfestigten Vliesen auch kalanderverfestigte. Diese entsprechen dem Stand der Technik. Die darin beschriebenen Vliese weisen daher die gen annten Nachteile auf.

Das Patent EP 2 008 565 A2 verfolgt die Idee ein Material mit maximaler Festigkeit und geringer Dehnung bereitzustellen. In der EP2008565 A2 wird Bindemittel-gebundenes Vlies beschrieben, was aufgrund dieser Verfestigungsart keine hohe Dehnfähigkeit besitzt.

Die WO 2008 / 066417 A1 beschreibt die Herstellung eines Spinnvliesstoffes, auf welches eine oder zwei Lagen von Kurzschnittfasern mit einer Stapellänge von 3 bis 7mm gelegt ist. Der Verbund der einzelnen Lagen geschieht mittels Wasserstrahlen. Zur Erhöhung der Abriebfestigkeit wird zusätzlich eine thermische Kalanderbehandlung durchgeführt. Das Material gemäß der WO 2008 / 066417 A1 ist daher auf maximale Festigkeiten ausgelegt.

Bezogen auf das vorerwähnte Anforderungsprofil ergibt sich, dass wasserstrahlverfestigte Vliesstoffe die Anforderungen a), c), d), e) und f) erfüllen, aber die anderen Anforderungen nicht.

Die kalanderverfestigten Vliesstoffe und auch die Bindemittel-verfestigten Vliese aus dem Stand der Technik erfüllen die Anforderungen b), d) und e), aber die anderen Anforderung nicht.

Die Aufgabe der vorliegenden Erfindung war es daher die vorbeschriebenen Nachteile des Standes der Technik zu vermeiden. Gelöst wird die Aufgabe durch die Merkmale des Anspruchs 1. Sinnvolle Ausgestaltungen sind in den Unteransprüchen 2 bis **4** genannt. Die Verwendung erfindungsgemäßer Materialien ist Anspruch **5** zu entnehmen.

Die im nachfolgenden Text verwendeten Begriffe und Testmethoden werden wie folgt definiert:

Trockenverfahren: bezeichnet das Herstellverfahren für erfindungsgemäße Deckschichten. Erfindungsgemäße Deckschichten können nach einem im Buch "Vliesstoffe", erschienen im Verlag Wiley VCH, 2. Auflage, 2012 aufgezeigten Trockenverfahren hergestellt werden. Erfindungsgemäß bevorzugt, aber ohne darauf beschränkt zu sein, wird das Kardierverfahren unter Verwendung von Stapelfasern. Die nachfolgende Beschreibung beschreibt die Erfindung anhand von mittels Kardierverfahren hergestellten Vliesstoffen.

Thermische Kalanderverfestigung: bezeichnet ein Verfestigungsverfahren für erfindungsgemäße Deckschichten. Die grundlegenden Techniken dazu werden im Buch "Vliesstoffe", erschienen im Verlag Wiley VCH, 2. Auflage, 2012, Seiten 385-395 beschrieben. Erfindungsgemäß bevorzugt, aber ohne darauf beschränkt zu sein, wird ein Thermobond-Kalander, d.h. ein mittels Druck- und Hitze arbeitendes System. Dabei wird eine Kombination aus glatter Walze und gravierter Gegenwalze, was eine punktweise Verschweißung der den Vliesstoff bildenden Fasern zur Folge hat, eingesetzt. Die Verwendung eines Ultraschallkalanders ist ebenso möglich. Kennzeichnend für Kalandergravuren sind die Verfestigungsfläche, auch als Pressfläche bezeichnet, und die Anzahl von Verfestigungspunkten pro Flächeneinheit, auch als Figurenanzahl bezeichnet. Die Verfestigungsfläche wird in % angegeben und beschreibt wie viel der Werkstoffoberfläche, im vorliegenden Fall der Vliesoberfläche, mit Verschweißungspunkten bedeckt ist. Die zum Einsatz kommenden Gravurmuster sind bevorzugt Ellipsen, andere Formen der Verfestigungspunkte wie beispielsweise Rauten, Stäbchen oder Kreise sind nicht ausgeschlossen. Die Verfestigungspunkte sind bevorzugt gleichmäßig auf der Walzenoberfläche verteilt. Anordnungen der Verfestigungspunkte, beispielsweise in Form von sich kreuzenden Verfestigungs-Punkt-Reihen mit größeren freien Flächen zwischen den Reihen sind ebenfalls vorstellbar.

Wasserstrahlverfestigung: bezeichnet ein Verfestigungsverfahren für erfindungsgemäße Deckschichten. Die grundlegenden Techniken dazu werden im Buch "Vliesstoffe", erschienen im Verlag Wiley VCH, 2. Auflage, 2012, Seiten 340-359 beschrieben.

Die Begriffe Faser und Stapelfaser werden im Rahmen dieser Beschreibung synonym verwendet. Sie bezeichnen synthetisch hergestellte Faserstoffe , die eine definierte Schnittlänge, angegeben in mm und einen definierten Faserdurchmesser, auch bezeichnet als Titer und in dtex angegeben, aufweisen. Die Fasern bestehen, sofern nicht explizit erwähnt, aus thermoplastischen Polymeren wie beispielsweise, und ohne darauf beschränkt zu sein, Polypropylen, Polyester, Polyamid, Polyethylen, Polylactid. Üblicherweise werden Fasern im Titerbereich von 1.0 dtex bis 4.4 dtex, wobei der Bereich von 1.3 bis 2.2 dtex erfindungsgemäß bevorzugt wird. Sollten Kardierverfahren zur Anwendung kommen, liegen die bevorzugten Schnittlängen im Bereich von 35 bis 55 mm, besonders bevorzugt bei 40 bis 50mm. Die Faser-Höchstzugkraftdehnungen einsetzbarer Fasern liegen im Bereich von 130 bis 580%.

Für die in Tabelle 1 genannten Materialien kamen folgende Fasermischungen und Verfahrenseinstellungen zum Einsatz:

Muster 1: sawatex 2626, 25 g/m$^2$

➤ Faser: 50Gew % Polypropylenfasern,1,3dtex/40mm, 50Gew% Polypropylenfasern 2.2dtex / 40mm,

➤ Spezifischer Energieaufwand bei der Wasserstrahlverfestigung: 0,157kW/kg

➤ Kalandereinstellung / Pressfläche / Figurenanzahl: keine, nur Wasserstrahlverfestigung

Muster 2: sawabond 4160, 25g/m$^2$

➤ Faser: 100% Polypropylenfasern, 2.2dtex / 40mm, Faser-Höchstzugkraftdehnung ca 560%

➤ Spezifischer Energieaufwand bei der Wasserstrahlverfestigung: keiner, da Kalanderverfestigung

➤ Kalandereinstellung / Pressfläche / Figurenanzahl: Druck 75daN/cm, 150°C, 11% Pressfläche, 25 Figuren / cm$^2$

Muster 3: sawatex 52DI020203, 25g/m$^2$

➤ Faser: 50Gew % Polypropylenfasern,1,3dtex/40mm, 50Gew% Polypropylenfasern 2.2dtex / 40mm,

➤ Spezifischer Energieaufwand bei der Wasserstrahlverfestigung: 0,057kW/kg

➤ Kalandereinstellung / Pressfläche / Figurenanzahl: keine, nur Wasserstrahlverfestigung

Muster 4: sawatex 25DI020401, 25g/m$^2$

➤ Faser: 50Gew % Polypropylenfasern,1,3dtex/40mm, 50Gew% Polypropylenfasern 2.2dtex / 40mm

➤ Spezifischer Energieaufwand bei der Wasserstrahlverfestigung: 0,057kW/kg

➤ Kalandereinstellung / Pressfläche / Figurenanzahl: Druck 75daN/cm, 150°C, 11% Pressfläche, 25 Figuren / cm$^2$

Muster 5: sawatex 60DI020402, 25g/m$^2$

➤ Faser: 50Gew % Polypropylenfasern,1,3dtex/40mm, 50Gew% Polypropylenfasern 2.2dtex / 40mm

➤ Spezifischer Energieaufwand bei der Wasserstrahlverfestigung: 0,057kW/kg

➤ Kalandereinstellung / Pressfläche / Figurenanzahl: Druck 75daN/cm, 150°C, 5% Pressfläche, 9 Figuren / cm$^2$

Des Weiteren fanden zur Ermittlung der in Tabelle 1 angegebenen Daten die nachfolgend genannten Prüfmethoden Verwendung.

Dabei ist zu beachten: wird die Bezeichnung "längs", "Längsrichtung" oder "MD" verwendet, so bezeichnet dies jeweils die Ausrichtung eines Muster in Fertigungsrichtung des Materials. Die Bezeichnungen "quer", "Querrichtung" oder "CD" bezeichnen die Ausrichtung eines Musters quer zu Fertigungsrichtung eines Materials. Soweit nicht anders angegeben, wurde bei Zugversuchen zur Simulation der Breite eines Windelohrs eine Probenbreite von 25mm bei einer Einspannlänge von 127mm gewählt.

- Flächengewicht: gemäß WSP 130.1, Angabe in g/m$^2$
- Dicke: gemäß WSP 120.6, ermittelt bei 0,5kPa Messdruck, Angabe in mm
- Maximalkraft Fmax Längs / Quer: gemäß WSP 110.4, Opt.A, Einspannlänge 127mm, Angabe in N/25mm
- Dehnung bei Fmax Längs / Quer: gemäß WSP 110.4, Opt.A, Einspannlänge 127mm, Angabe in %
- Dehnung bei 5N Längs / Quer: gemäß WSP 110.4, Opt.A, Einspannlänge 127mm, Angabe in %, automatische Ermittlung der Dehnung bei Erreichen einer Kraft von 5N im Zugversuch
- Faser-Bruchdehnung: gemäß ISO 5079, Angabe in %

- Breiteneinsprung: dieser wird als Verhältnis der Breite B0 einer Vliesbahn im unbelasteten Zustand flach auf einem Tisch liegend zu der Breite B1 derselben Vliesbahn ermittelt, wenn diese mit einem Zug von 10N belastet wird. Zur Ermittlung der Breite unter Belastung wird ein 150 cm langes und 20cm breites Vliesmuster zunächst am oberen Ende eines Testaufbaus fixiert, sodass ein mindestens 120 langes Vliesstück frei herabhängen kann. Die Fixierung findet dabei so statt, dass die Vliesbahn über die komplette Breite gehalten wird. Nun wird im Abstand von 110 cm zu oberen Klemmung ein über die komplette Bahnbreite fixierbares Massestück angebracht, sodass sich an der Vliesbahn eine Belastung von 10N/20cm ergibt. Im Abstand von 55cm zur oberen Klemme wird dann erneut die Breite B1 der Vliesbahn ermittelt. Der Breiteneinsprung ergibt sich dann nach der folgenden Formel:

$$\text{Breiteneinsprung (\%)} = \frac{B1}{B0} * 100\%$$

- Für die Zwecke der vorliegenden Erfindung wird der spezifische Energieaufwand gemäß den im Buch "Vliesstoffe", Viley VCH, 2012 auf Seite 343 angegebenen Formeln 6.30 und 6.31 errechnet und in kW/kg angegeben.

Vliesstoffe, die als Deckmaterialien für den genannten Einsatzzweck geeignet sind, . weisen im allgemeinen Flächengewichte auf, die im Bereich zwischen 20 und 40 g/m² liegen. Unterhalb 20 g/m² sind die mechanischen Festigkeiten zu gering, Flächengewichte von 40g/m² und höher sind einerseits wirtschaftlich nicht interessant, andererseits bauen derartige Vliese höhere Kräfte auf, sodass diese nicht mehr für den Einsatzzweck geeignet sind. Sie bestehen zumeist aus nach Trockenverfahren hergestellten Vliesstoffen aus Stapelfasern, welche thermisch mittels Kalandern verfestigt werden.

Betrachtet man die bei Zugbelastung eines derartigen Vlieses in Querrichtung aufgezeichnete Kurve in Figur1, Kurve 2, so kann man erkennen, dass kalanderverfestigte Vliesstoffe einen mehr oder weniger linearen Kraftanstieg im Bereich von 0 bis 100% Vliesdehnung aufweisen. Grund dafür ist das Vorliegen von starren Verbindungspunkten.

Bei der Herstellung eines Stapelfaservlieses nach einem Trockenverfahren wie beispielsweise dem Kardieren werden einzelne Faserflorschichten übereinander gelegt. Faserkreuzungspunkte bestehen nur an direkt aufeinander liegenden Einzelfasern, wobei dabei keine Bindungskräfte aufgebaut werden. Der so hergestellte, unverfestigte Faserflor muss daher einer Verfestigung unterzogen werden.

Führt man einen derartigen Faserflor einer thermischen Kalanderverfestigung zu, werden durch die Kompression im Kalanderspalt nur die tatsächlich übereinander liegenden Fasern miteinander verschmolzen. Die Verschmelzung findet auch nur dort statt, wo ein Verfestigungspunkt auf der gravierten Kalanderwalze die Fasern aus dem Faserflor trifft und auf die glatte Kalanderwalze drückt und, bedingt durch Druck und Hitze miteinander verschweißt. Die so ausgebildeten Verfestigungspunkte sind, da thermisch ausgebildet, starr und im Vliesverbund unbeweglich. Ein so hergestelltes Vlies hat eine gute Dimensionsstabilität bei der Weiterverarbeitung. Der Breiteneinsprung bei Zugbelastung in Längsrichtung liegt bei ca 3%, das Vlies ist gegenüber Bahnzugschwankungen unempfindlich. Vergleiche hierzu Tabelle1, Muster 2.

Das Dehnverhalten derartiger Vliesstoffe kann in Querrichtung beispielsweise durch die Wahl der verwendeten Fasern beeinflusst werden. Fasern mit höherer Faserdehnung bringen bei gleichen Verfestigungseinstellungen auch eine höhere Dehnung in Querrichtung. Andererseits kann man beispielsweise auch durch die Wahl der Kalandergravur Einfluss nehmen. Eine Verringerung der Figurenanzahl pro cm² bei gleicher insgesamter Verfestigungsfläche führt zur Erhöhung der Dehnung, allerdings nimmt dadurch auch die Festigkeit ab. In der Folge ist ein solches Material mechanisch so instabil, dass eine sichere Verarbeitung nicht mehr möglich ist, der Breiteneinsprung und das Abflusen sind zu hoch.

In einem Windelohr liegt die Vliesbreite des der Zugbelastung ausgesetzten Bereichs zwischen 20 und 40mm. Wie eingangs erwähnt, liegt die Zugbelastung, die ein Benutzer beim Schließen einer Windel aufbaut, im Bereich bis maximal 10N. Üblicherweise werden Elastiklaminate beidseitig mit Deckmaterialien abgedeckt. Daraus ergibt sich eine Belastung für eine einzelne Lage Deckmaterial von maximal 5N/Windelohrbreite. Die Belastung, die aufgebaut wird, liegt also in einem Bereich von ca 5N/30mm. Dies stellt eine Kraft dar, bei welcher ein kardiertes und thermisch kalanderverfestigtes Vlies bereits gerissen, zumindest aber mechanisch stark geschädigt ist. Vergleiche hierzu Tabelle1, Muster 2.

Wird also ein so hergestelltes und verfestigtes Vlies in einem Windelohr ohne weitere Vorbehandlung verwendet, kommt es zu einer unkontrollierten Zerstörung des Vlieses während der Verwendung. Daher und um auch die Steigung der Kraft-Dehnungskurve zu verringern, wird üblicherweise eine kontrollierte Vorschädigung des Vlieses im laminierten Zustand vorgenommen. Dieser Vorgang wird als Aktivierung bezeichnet.

Bei einem Aktivierungsvorgang, beispielweise mittels sogenanntem Ringrolling, werden die Bindepunkte über die gesamte der Aktivierung unterworfene Fläche aufgebrochen. In der Folge ist die Integrität des Vlieses zerstört. Es können beispielsweise Fasern abflusen.

Der Trend bei der Herstellung Windelohren geht daher zu Verfahren, die einen Aktivierungsgang vermeiden, d.h. die zum Einsatz kommenden Deckmaterialien müssen bereits die geforderte geringe Steigung der Kraft-/Dehnungskurve aufweisen, sodass die eingangs erwähnten Anforderungen in einem Deckmaterial vereinigt sind.

Die Kurve 1 in Figur 2 zeigt das Kraft-Dehnungsverhalten eines wasserstrahlverfestigten Vlieses bei Belastung in Querrichtung. Am Beginn der Belastung zeigt sich im Vergleich zu kalanderverfestigten Vliesen eine geringere Steigung der Kurve, die dann aber bei Überschreiten einer Dehnung von ca 100% fast exponentiell ansteigt. Dies wird erklärt durch eine Knotenbildung unter den einzelnen Fasern in den Faserverwirbelungspunkten. Dieser Effekt ist für die Anwendung positiv zu sehen, der Verwender nimmt dieses Verhalten am Windelohr ebenso war, dadurch wird einem Überdehnen in der Verwendung vorgebeugt.

Die Faserverwirbelungen sind in einem wasserstrahlverfestigten Vlies nicht starr fixiert sondern in sich beweglich, da die Fasern dreidimensional miteinander verschlungen aber nicht verschweißt sind. Die Faserverwirbelungen liegen dabei über die gesamte Fläche des Vliesstoffes gleichmäßig vor.

Wirkt eine mechanische Belastung auf ein derartiges Vlies, so können sich die miteinander verwirbelten Fasern über einen weiten Teil dieser Belastung anpassen, ohne dass sich die Faserverwirbelungen auflösen und die strukturelle Integrität des Vlieses verloren geht.

Andererseits aber sind flexible Faserverwirbelungspunkte in der Herstellung und Weiterverarbeitung durch die damit zusammenhängende hohe Empfindlichkeit derartiger Vliese gegen in Längsrichtung wirkenden Zugbelastungen nachteilig. Zugbelastungen in Längsrichtung führen zu Breiteneinsprüngen, die teilweise über 20% betragen, vergleiche Tabelle 1, Muster 1.

Die Intensität der Faserverwirbelungen ist von der Menge der für die Verfestigung aufgewandten Energie abhängig. Je höher der Energieaufwand ist, umso stärker sind die Fasern mit einander verwirbelt. Im Rahmen der vorliegenden Beschreibung wird die für die Verfestigung eingesetzte Energie als spezifischer Energieaufwand ermittelt und angegeben. Dieser Wert berücksichtigt alle für die Verfestigung notwendigen Einstellungen / Anlagenteile und gewährleistet so die Vergleichbarkeit des Energieaufwands bei unterschiedlichen Anlagenkonfigurationen

Für die vorliegende Erfindung wird daher der spezifische Energieaufwand aus der Anzahl verwendeter Wasserstrahlbalken, dem Düsendurchmesser, der Anzahl Düsenbohrungen, dem Druck im Wasserstrahlbalken, der Arbeitsbreite, der Maschinengeschwindigkeit und des Flächengewichts errechnet und in kW/kg angegeben. Die notwendigen Formeln dazu sind im Buch "Vliesstoffe", Viley VCH, 2012 auf Seite 343 unter 6.30 und 6.31 angegeben. Die Ermittlung des spezifischen Energieaufwands hat den Vorteil, dass dieser auch ermittelt werden kann, wenn nur eine geringe Verfestigung stattfindet, die ein Produkt zur Folge hat, welches mechanisch instabil ist, daher weder Festigkeiten o.ä. ermittelbar sind.

Der vorliegenden Erfindung liegt daher die Idee zugrunde, die flexiblen Faserverwirbelungen durch das Einbringen von starren Bindepunkten zu ergänzen um dadurch die Vorteile beider Technologien zu nützen.

In einem erfindungsgemäß ausgeführten Vliesstoff, der für Deckmaterialien einsetzbar ist, liegen daher beide Bindungsarten nebeneinander vor.

Nach der Herstellung eines unverfestigten Faserflors, beispielsweise nach dem Kardierverfahren, werden in einem ersten Verfestigungsschritt durch das Wasserstrahlverfahren flexible Faserverwirbelungen erzeugt und in einem zweiten Verfestigungsschritt durch thermische Verschweißung partiell fixiert, sodass ein Teil der flexiblen Faserverwirbelungen in starre Verbindungspunkte überführt wird.

Dadurch wird die mechanische Stabilität des Vliesstoffes in Längsrichtung verbessert, ohne dass sich die Eigenschaften bei Querdehnung im funktionellen Bereich merklich verschlechtern.

Ein dem Stand der Technik entsprechendes Deckmaterial wie Muster 1 wird mit einem spezifischen Energieaufwand von 0,157kW/kg verfestigt.

Führt man nun eine Kombination der beiden Verfahren Wasserstrahl- und Kalanderverfestigung nach dem Stand der Technik durch, so erzielt man, wenn man die Einstellungen aus Muster 1 und 2 einfach kombiniert , ein Material welches eine sehr steile Kraft-Dehnungskurve aufweist. Es ist daher für den gedachten Einsatz ungeeignet.

Erfindungsgemäß werden daher die einzelnen Verfestigungsverfahren so weit in ihrer Intensität reduziert, dass Vliesstoffe, die jeweils mit nur einem der beiden Verfahren verfestigt werden, nicht mehr mechanisch stabil sind. Das Muster 3 wurde beispielsweise nur mittels Wasserstrahlen verfestigt. Der spezifische Energieaufwand lag bei 36,3% dessen von Muster 1.

Wie man aus der Tabelle 1 für das Muster 3 und auch der Figur 1, Kurve 3 entnehmen kann, hat ein geringer spezifischer Energieaufwand bei der Wasserstrahlverfestigung ein mechanisch instabiles Vlies zur Folge, der Breiteneinsprung beträgt knapp 40%, ein derartiges Material ist daher nicht verarbeitbar.

Beim Muster 3a wurde ein Faserflor analog zu Muster 2 hergestellt und mit einem Kalander analog zu Muster 5 verfestigt. Wie Tabelle 1 zu entnehmen ist, weist das Muster keinerlei mechanische Stabilität auf.

Unterzieht man Muster 3 einer weiteren thermischen Verfestigung mittels Kalander und setzt dabei die gleiche Gravur wie bei Muster 2 ein, d.h. 25 Verfestigungspunkte/ cm$^2$ bei einer Gesamtverfestigungsfläche von 11%, so ergibt sich Muster 4.

Wie man aus Tabelle 1 erkennen kann, hat Muster 4 mit 5,5N/25mm eine gerade noch für den Anwendungszweck ausreichende Querfestigkeit, jedoch ist die Dehnung bei 5N Belastung mit 106% im Vergleich zu Muster 1 mit 123% schlechter. Der Breiteneinsprung mit 7,9% ist als gut zu betrachten. Betrachtet man in Figur 1 die dem Muster 4 entsprechende Kurve 4, so kann man auch leicht erkennen, dass die Steigung der Kurve im Vergleich zu den Mustern 1 bis 3 und 5 deutlich steiler ist. Das Muster 4 reißt auch bereits kurz nach dem Erreichen einer Kraft von 5N. Es ist daher nicht für den Einsatzzweck als Deckmaterial geeignet.

Nimmt als Basismaterial wiederum ein Material gemäß Muster 3 und verfestigt dieses mittels eines Kalanders, dessen Verfestigungsfläche 5% beträgt und 9 Verfestigungspunkte pro $cm^2$ aufweist, so kommt man zu Muster 5.

Muster 5 hat im Vergleich zu Muster 4 eine um 50% reduzierte Verfestigungsfläche. Trotzdem ist die Längsfestigkeit auf gleichem Niveau, betrachtet man die Querfestigkeit, so ist diese gegenüber Muster 4 leicht erhöht.

Deutlicher wird der Unterschied von Muster 5 zu Muster 4 in Bezug auf das Kraft-Dehnungsverhalten. Die Kraft-Dehnungskurve von Muster 5 ist in Figur 1 als Kurve 5 gezeigt. Sie zeigt sich ideal für den erfindungsgemäßen Einsatzzweck als Deckmaterial. Im Bereich bis 100% Dehnung wird fast keine Kraft aufgebaut, im Bereich über 100% Dehnung steigt die Kraft rasch an, sodass sich die gewünschte Stopp-Funktion ergibt. Muster 5 weist eine Fmax-Dehnung in Querrichtung in Höhe von 163% auf und liegt damit um 53% höher als Muster 4, ist auf dem Niveau der Muster 1 und 2.

Betrachtet man das Verhalten von Muster 5 im Bereich bis 5N, so zeigt sich eine im Vergleich zu Muster 4 immer noch um 50% höhere Dehnung bei 5N. Diese ist auch gegenüber dem Stand der Technik, Muster 1 noch um 30% höher. Ein Vergleich mit Muster 2 ist nicht möglich, da dies bereits bei einer Belastung von 2,7N/25mm gerissen war.

Bezüglich des Breitensprunges bei der Verarbeitungsspannung von ca 10N in Längsrichtung zeigt sich, dass das Muster 5 mit einem Breiteneinsprung von 3,5% nahe dem von Muster 2 zu liegen kommt und daher um das knapp 6-fache geringer im Vergleich zu Muster 1.

Auch die Dicke von Muster 5 ist durch die Kombination von Wasserstrahlverfestigung und Kalanderverfestigung geringer als bei Muster 1 oder 2. Dies ist vorteilhaft, da sich dadurch bei gleichem Rollendurchmesser mehr Laufmeter aufwickeln lassen. In der Folge kommt es zu weniger Rollenwechseln pro Zeiteinheit während der Verarbeitung und dadurch bedingt auch zu geringen Abfallanteilen.

Die beiden bei Muster 5 erfindungsgemäß nacheinander durchgeführten Verfestigungsschritte Wasserstrahlvernadelung und thermische Kalanderbehandlung entsprechen nur vom prinzipiellen Verfahrensablauf den im Stand der Technik genannten Verfahren. Erfindungsgemäß sind die einzelnen Verfestigungsverfahren so weit in ihrer Intensität reduziert, sodass Vliesstoffe, die jeweils mit nur einem der beiden Verfahren verfestigt werden, mechanisch nicht ausreichend stabil sind. Vergleiche hierzu Muster 3 und 3a aus Tabelle 1.

Für die Herstellung des erfindungsgemäß ausgeführten Musters 5 wurden der spezifische Energieaufwand und auch die Verfestigungsfläche am Kalander wie aus Tabelle 1 erkennbar deutlich reduziert.

Vergleicht man beispielsweise den spezifischen Energieaufwand bei der Herstellung eines erfindungsgemäß ausgeführten Materials 5 zu Muster 1 so beträgt dieser nur 36 % dessen von Muster 1. Auch die angewandte Verfestigungsfläche am Kalander liegt nur bei 45% im Vergleich zum Stand der Technik gemäß Muster 2. Aus den Ergebnissen von Muster 4 im Vergleich zu Muster 5 kann man erkennen, dass trotz deutlich unterschiedlicher Verfestigungsfläche die Festigkeiten auf nahezu gleichem Niveau liegen. Der Verfestigungsflächenanteil von 11% wird daher als Obergrenze angesehen. Erfindungsgemäß bevorzugt werden Verfestigungsflächen von kleiner 8% und besonders bevorzugt von kleiner gleich 6%.

Aber nicht nur die Verfestigungsfläche, auch die Anzahl der Verfestigungspunkte ist für die Ausbildung eines erfindungsgemäßen Vliesstoffes wichtig. Erfindungsgemäß geeignet sind Gravuren, die nur wenige Verschweißungspunkte/ $cm^2$ ausbilden. Erfindungsgemäß bevorzugt werden Gravuren die entsprechend Muster 5 ausgeführt sind. Sie sollten weniger als 10 Verbindungspunkte/$cm^2$ aufweisen, wobei Gravuren mit weniger als 7 Verbindungspunkte/ $cm^2$ besonders bevorzugt sind. Die Fläche eines einzelnen Verbindungspunktes sollte dabei mehr als 0,65$mm^2$, bevorzugt 0,70$mm^2$ betragen. Die gewährleistet das Vorhandensein einer geringen Anzahl von Verschweißungen, die aber durch ihre im Vergleich beispielsweise zur Gravur von Muster 2 mit einer Fläche eines Gravurpunkts von 0,44$mm^2$ eine deutlich größere Bindepunktfläche ausbilden. Dadurch wird im Verschweißungspunkt eine Vielzahl von Faserverwirbelungen erfasst und das so entstandene Vlies stabilisiert.

Derart erfindungsgemäß ausgeführte Vliesstoffe sind als Deckmaterialien für elastische Verschlusssysteme besonders geeignet, da diese im Vergleich zum Stand der Technik alle Punkte des eingangs genannten Anforderungsprofils erfüllen.

Darüber hinaus sind diese mit einem im Vergleich zum Stand der Technik deutlich geringeren Energieaufwand herstellbar und leisten somit einen positiven Beitrag zur CO2-Bilanz der Industrie.

Bezugszeichenliste:

1 - Kraft-Dehnungs-Kurve zu Muster 1
2 - Kraft-Dehnungs-Kurve zu Muster 2
3 - Kraft-Dehnungs-Kurve zu Muster 3
4 - Kraft-Dehnungs-Kurve zu Muster 4
5 - Kraft-Dehnungs-Kurve zu Muster 5

| Tabelle 1 | Einheit | Muster 1 | Muster 2 | Muster 3 | Muster 3a | Muster 4 | Muster 5 |
|---|---|---|---|---|---|---|---|
| Verfestigungsart | | wasserstrahl | thermisch kalander | wasserstrah | thermisch kalander | wasserstrah + thermisch kalander | wasserstrahl + thermisch kalander |
| Energieeintrag bei Wasserstrahlverfestigung | kW/kg | 0,157 | ---- | 0,057 | ---- | 0,057 | 0,057 |
| Verfestigungsfläche am Kalander | % | ---- | 11 | ---- | 5 | 11 | 5 |
| Anzahl Verfestigungspunkte | Punkte / cm$^2$ | ---- | 25 | ---- | 9 | 25 | 9 |
| Fläche eines Verfestigungspunktes | mm$^2$ | ---- | 0,44 | ---- | 0,72 | 0,44 | 0,72 |
| Flächengewicht | g/m$^2$ | 25,1 | 26,4 | 24,8 | n.m. | 25,0 | 26,4 |
| Dicke | mm | 0,56 | 0,49 | 0,60 | n.m. | 0,39 | 0,40 |
| Fmax MD | N/ 25mm | 24,0 | 19,9 | 11,5 | n.m. | 27,7 | 26,5 |
| Fmax CD | N/ 25mm | 8,5 | 2,7 | 1,4 | n.m. | 5,5 | 5,8 |
| Breiteneinsprung | % | 21,1 | 3,0 | 43,1 | n.m. | 7,9 | 4,3 |
| Dehnung bei Fmax MD | % | 52,7 | 73,3 | 49,0 | n.m. | 32,5 | 28,0 |
| Dehnung bei Fmax CD | % | 147,6 | 158,4 | 149,5 | n.m. | 110,6 | 163,0 |
| Dehnung bei 5N CD | % | 123,1 | Z | Z | n.m. | 106,4 | 155,2 |
| Dehnung bei 5N MD | % | 17,05 | 7,9 | 26,5 | n.m. | 7,0 | 3,7 |
| Z= vor Erreichen des Bezugspunktes zerstört<br>n.m.= nicht messbar | | | | | | | |

EP 3 000 451 B1

**Patentansprüche**

1. Dehnbarer Vliesstoff aus thermoplastischen Stapelfasern
**dadurch gekennzeichnet, dass**

➢ der Vliesstoff aus Fasern besteht, deren Fasertiter im Bereich von 1.0 bis 4.4 dtex liegt und deren Schnittlänge 35-55mm betragen,

➢ der Vliesstoff starre, als Verschweißungen und flexible, als Faserverwirbelungen ausgebildete Verbindungspunkte aufweist,

➢ die Faserverwirbelungen durch Wasserstrahlvernadelung erzeugt sind,

➢ die Verschweißungen durch eine Behandlung mittels Kalanders erzeugt sind,

➢ der Anteil der Verschweißungen nicht mehr als 11 % der Vliesoberfläche beträgt,

➢ der Anteil von Verschweißungspunkten kleiner 10 pro $cm^2$ liegt und

➢ ein Verschweißungspunkt eine Fläche von mindestens 0,65$mm^2$ aufweist

2. Dehnbarer Vliesstoff nach einem der vorhergehenden Ansprüche,
**dadurch gekennzeichnet, dass**
der Anteil von Verschweißungen nicht mehr als 8 % der Vliesoberfläche beträgt.

3. Dehnbarer Vliesstoff nach einem der vorhergehenden Ansprüche,
**dadurch gekennzeichnet, dass**
der Anteil von Verschweißungen nicht mehr als 6 % der Vliesoberfläche beträgt.

4. Dehnbarer Vliesstoff nach einem der vorhergehenden Ansprüche,
**dadurch gekennzeichnet, dass**
der Vliesstoff nicht mehr als 7 Verschweißungspunkte pro $cm^2$ aufweist.

5. Elastisches, mehrlagiges Laminat zur Verwendung in einem Verschlusssystem für persönliche Hygieneartikel, bestehend aus einem elastischen Basismaterial, ein- oder beidseitig abgedeckt mit einem Deckmaterial,
**dadurch gekennzeichnet, dass**
das Deckmaterial aus einem dehnbaren Vliesstoff entsprechend der Ansprüche 1 bis **4** gebildet wird.


**Claims**

1. Stretchable fleece material of thermoplastic staple fibres **characterized in that**

➢ the fleece material is made of fibres whose titre lies in the range of 1.0 to 4.4 dtex, and whose cut lengths are 35-55 mm,

➢ the fleece material has stiff connecting points in the form of welds, and flexible connecting points in the form of fibre entanglement,

➢ the fibre entanglements are produced by hydroentanglement,

➢ the welds are produced by a calender treatment,

➢ the proportion of welds is no more than 11 % of the surface of the fleece,

➢ the proportion of weld points is less than 10 per $cm^2$, and

➢ a weld point has an area of at least 0.65 $mm^2$.

2. Stretchable fleece material according to one of the preceding claims,
**characterized in that**
the proportion of welds is no more than 8% of the surface of the fleece.

**3.** Stretchable fleece material according to one of the preceding claims,
**characterized in that**
the proportion of welds is no more than 6% of the surface of the fleece.

**4.** Stretchable fleece material according to one of the preceding claims,
**characterized in that**
the fleece material has no more than 7 weld points per cm$^2$.

**5.** Elastic, multilayer laminate for use in a closure system for personal hygiene products, consisting of an elastic base material covered on one or both sides with a covering material,
**characterized in that**
the covering material is made of a stretchable fleece material according to claims 1 through 4.


**Revendications**

**1.** Tissu non tissé extensible à base de fibres discontinues thermoplastiques **caractérisé en ce que**

➤ le tissu non tissé est constitué de fibres, dont le titre de fibre se situe dans la plage de 1,0 à 4,4 dtex et dont les longueurs vont de 35 à 55 mm,

➤ le tissu non tissé présente des points de liaison se développant sous forme de points rigides en forme de soudures et de points souples en forme d'enchevêtrements de fibres,

➤ les enchevêtrements de fibres sont créés par un aiguilletage au jet d'eau,

➤ les soudures sont créées par un traitement au moyen d'une calandre,

➤ la proportion des soudures n'est pas supérieure à 11 % de la surface du non tissé,

➤ la proportion des points de soudure se situe inférieure à 10 par cm$^2$ et

➤ un point de soudure présente une surface d'au moins 0,65 mm$^2$.

**2.** Tissu non tissé extensible selon l'une des revendications précédentes,
**caractérisé en ce que**
la proportion des soudures n' st pas supérieure à 8 % de la surfac de non tissé.

**3.** Tissu non tissé extensible selon l'une des revendications précédentes,
**caractérisé en ce que**
la proportion des soudures n'est pas supérieure à 6 % de la surface de non tissé.

**4.** Tissu non tissé extensible selon l'une des revendications précédentes,
**caractérisé en ce que**
le tissu non tissé ne présente pas plus de 7 points de soudure par cm$^2$.

**5.** Stratifié élastique, en plusieurs couches, destiné à l'emploi dans un système de barrage pour des articles d'hygiène personnelle, constitué d'une matière de base élastique, recouverte sur un côté ou sur les deux côtés avec un revêtement,
**caractérisé en ce que**
le revêtement est formé à base d'un tissu non tissé extensible correspondant aux revendications 1 à 4.

**Figur 1 :**

Fmax CD $\left[\dfrac{N}{25mm}\right]$

Dehnung Fmax CD [%]

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 2069141 B1
- EP 1921192 B1
- WO 03007864 A1
- EP 2008565 A2
- WO 2008066417 A1

**In der Beschreibung aufgeführte Nicht-Patentliteratur**

- **DR.HORNFECK ; BERNHUBER.** Performance and Fit. 2013, 56-62
- Vliesstoffe. Verlag Wiley VCH, 2012
- Vliesstoffe. Verlag Wiley VCH, 2012, 385-395
- Vliesstoffe. Verlag Wiley VCH, 2012, 340-359
- Vliesstoffe. Viley VCH, 2012, 343